# EUROPEAN PATENT SPECIFICATION

(11) **EP 0 560 950 B1**
(45) Date of publication and mention of the grant of the patent: **03.02.1999**
(21) Application number: 92917431.6
(22) Date of filing: 17.04.1992
(51) Int. Cl.: A61M 25/06

(54) **CANNULA**
KANÜLE
CANULE

(30) Priority: 19.04.1991 US 687843
(43) Date of publication of application: 22.09.1993
(73) Proprietor: BIOTIME, INC., Berkeley, CA 94170 (US)
(72) Inventor: SEGALL, Paul, E., Berkeley, CA 94708 (US); STERNBERG, Hal, Berkeley, CA 94708 (US); WAITZ, Harold, D., Berkeley, CA 94704 (US); COHEN, Bruce, Richmond, CA 94804 (US)
(74) Representative: Bizley, Richard Edward
(86) International application number: US9203239
(87) International publication number: WO9218174

(56) References cited:
- FR-A- 2 089 195
- US-A- 3 225 762
- US-A- 3 788 119
- US-A- 4 411 657
- US-A- 4 413 993
- US-A- 4 565 545
- US-A- 4 588 398
- US-A- 4 610 671
- US-A- 4 808 156
- US-A- 4 808 170
- US-A- 4 917 670
- US-A- 4 994 036

## Description

The present invention relates to the field of surgical devices.

Cannulas are hollow tube instruments used to deliver fluids or remove fluids from blood vessels, ducts or other hollow organs of animals. While many sizes of cannulas are available commercially, microcannulas suitable for use in surgery on small animals are of limited design and utility. The smallest available cannulas are generally flat tipped and are large enough to accommodate a 14 to 24 gauge (1.71 to 0.56mm) needle in the lumen of the cannula, which needle is used as a trochar. Small cannulas are generally made of small bore polyethylene tubing and are supplied with a hypodermic needle which is used to block a cannula to prevent fluid contained in the vessel of the hollow organ from draining until the cannula is in place in a pre-made incision in the vessel or other hollow organ.

As cannulas decrease in size, they are more flexible and easily bent and therefor difficult to manipulate. The flexibility of small cannulas occurs because of the decreasing absolute wall thickness of the cannula as they get smaller in diameter, and concomitant loss of rigidity of the cannula wall.

Conventional cannulas are supplied with trochars that move freely in the lumen of the cannula since it is conventionally desirable to be able to quickly remove the trochar once a vessel is cannulated.

Conventional small cannulas appropriate for use in cannulation of small blood vessels in microsurgery are notoriously difficult to use. The smallest cannulas available frequently require many minutes of patient and skilled manipulation to prepare a micro-incision in a blood vessel and properly place the cannula in small blood vessels.

US-A-3,788,119 (Baxter Laboratories, Inc) discloses a procedure for forming a bevelled tip on a needle assembly including a cannula and an obturator. The procedure provides an assembly having no appreciable clearance or mismatch between cannula and obturator, thereby to substantially reduce incidence of coring of tissue being punctured.

The present invention provides a microcannula comprising a hollow tube having a cross sectional size smaller than a 24 gauge(0.56 mm) needle and a bevelled tip wherein the angle of said bevel ranges from 23 to 27°, characterised in that the microcannula further comprises a pointed trochar that fits in the lumen of the microcannula tube.

In preferred embodiments of the present invention said bevel is 25° ± 0.50°. The outside diameter of said tube is preferably 0.4064 mm ± 0.0254 mm (0.016 inch ± 0.001 inch) and/or the inside diameter of said tube is preferably 0.2032 mm ± 0.0254 mm (0.008 inch ± 0.001 inch).

The walls of said tube may be a bio-compatible polymer, preferably a perfluorocarbon material.

Preferably the point of said trochar forms an angle of 8° ± 3° and/or the point of said trochar is six times the diameter of said trochar.

The outside diameter of said trochar is preferably smaller than the inside diameter of said tube, more preferably said trochar is removable from said microcannula but fits tightly in the lumen of said microcannula. Said trochar may be made of stainless steel.

The microcannula may further comprise a segment distal to the tip of said microcannula with an expanded outside diameter. The expanded outside diameter of said segment is preferably provided by a ring around the outside of said tube.

The above described microcannulas of the present invention therefore comprise a very small bore cannula having a bevelled tip with a sharp pointed trochar tightly, but removably placed in the lumen of the cannula. This cannula or microcannula and associated trochar have sufficient rigidity to be relatively easily manipulated. Furthermore, by means of using a sharp pointed trochar and bevel tipped cannula, it is advantageously possible to cannulate a small blood vessel without the necessity of incising the blood vessel wall before inserting the cannula and trochar.

The microcannula of the invention is easily manipulatable and may be used to cannulate small hollow organs and blood vessels in a short period of time. A benefit of the present invention is that the microcannula and trochar function together as a unit to provide rapid cannulation with a minimum of blood loss. A further benefit is that the microcannula and trochar may also be used to cannulate a blood vessel without making a preparatory incision before inserting the trochar and cannula into the blood vessel.

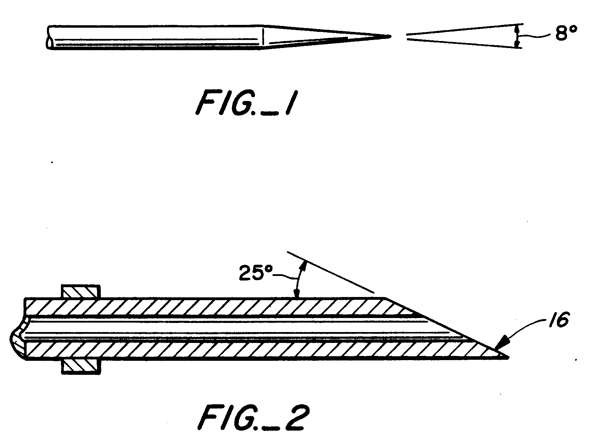

Figure 1 is a side view of the trochar according to the invention.
Figure 2 is a cross-section of the microcannula according to the invention.
Figure 3 shows the trochar and microcannula in use as a unit just prior to penetration of a blood vessel wall or other hollow tube by the point of the trochar.
Figure 4 shows the microcannula in place in a blood vessel or other hollow tube with the trochar in the process of being withdrawn.
Figure 5 shows the microcannula in place in a blood vessel or other hollow tube with the blood vessel tied off around the proximal end of the microcannula and the microcannula secured in place.

In greater detail, the microcannula according to the invention comprises a hollow tube 12 having a cross sectional size smaller than a 24 gauge (0.56 mm) needle. More precisely the outside diameter of said tube which comprises the body of the microcannula is about 0.4064 mm (0.016 inch). The outside diameter of the tubing will vary slightly but in general the outside diameter of the tube will be between 0.457 and 0.3556 mm (0.018 and 0.014 inch). Usually the outside diameter of the tubing will be 0.4046 mm ± 0.0754 mm (0.016 inch ± 0.001 inch).

The microcannula according to the invention will have a simple bevelled tip. The angle of said bevel is about 25°. The angle of the bevel may vary between 23 and 27 degrees, but the best performance of the microcannula is achieved when the bevel is 25° ± 0.5°.

The microcannula of the invention will generally be made of bio-compatible polymer tubing. It is preferred that the bio-compatible polymer be perfluorocarbon material.

The microcannula described above is highly flexible and delicate and is difficult to insert into the lumen of a blood vessel 18 or other hollow organ requiring cannulation. In order to facilitate manipulation of the microcannula, a trochar 10 is provided for use with the microcannula. The trochar fits in the lumen of the microcannula tube and may be removed therefrom. It is preferred that the trochar be of a size that fits tightly in the lumen of the microcannula tube and does not move freely in the lumen; however, the trochar must also be small enough to be removed from the lumen of the microcannula when the side of the microcannula is grasped and held and the trochar is pushed or pulled from the lumen of the tube. The preferred performance of the trochar is best obtained when the outside diameter of the trochar is slightly smaller than the inside diameter of the tube.

The trochar used with the cannula is pointed and the point forms an angle of about 8°. The angle of the point of said trochar may vary between about 11° and 5°. An angle of 8° is preferred. The length of the point of the trochar is about six times the diameter of said trochar. Lengths substantially greater that about six times the diameter of the trochar lead to undesirable delicate points that can flex and break. Therefore, it is preferred that the length of the trochar point is about six times the diameter of the trochar or less.

The trochar may be made of any strong wire stock. It is preferred that the wire is not of a ductile metal since the trochar confers rigidity on the microcannula when it is inserted into the lumen thereof. Furthermore ductile wires cannot be easily inserted into the lumen of the microcannula with the required tight fit without bending or breaking. It is preferred that the trochar is made of stainless steel.

The microcannula of the invention further comprises a segment of the microcannula tube located distal to the tip of the microcannula that has an expanded outside diameter. The expanded outside diameter of shoulder 14 may be in the form a ring of tubing or an "O" ring adhered to the outside wall of the microcannula tube, dried plastic glue or a thickening in the wall for the microcannula itself.

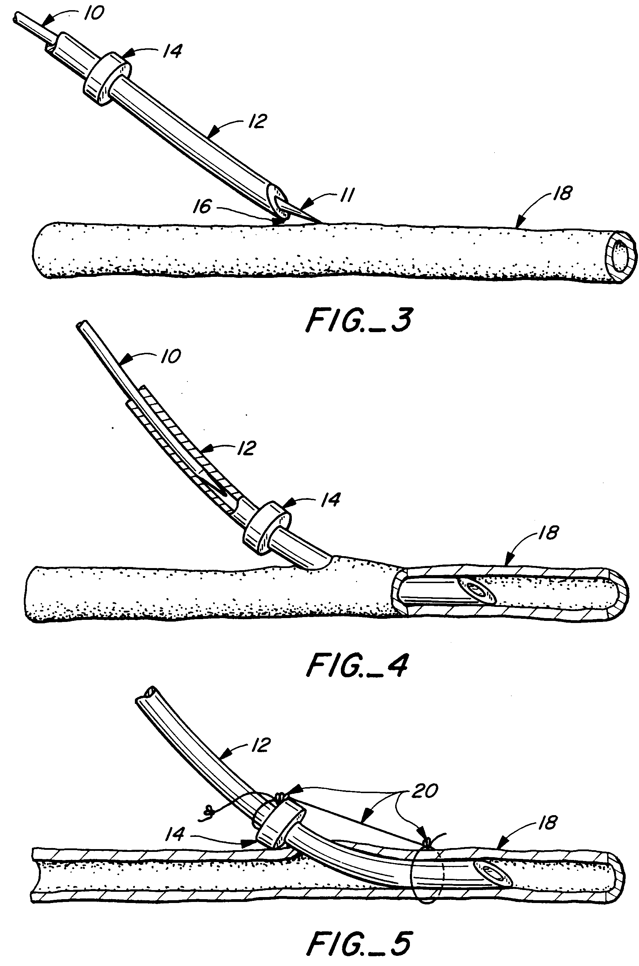

When in use, the shoulder is used to secure the distal end of the microcannula using a ligature 20, one end of which is tied around the distal end of the microcannula and the other end of which is tied around the blood vessel 18 surrounding the end of the microcannula proximal to the beveled tip, as depicted in Figure 5.

The distal end of the microcannula, which is the end of the microcannula away from the beveled tip, may be secured optionally within the lumen of a larger tube which may in turn be secured to the tip of a needle or still larger piece of tubing. The distal end of the microcannula can in this fashion be conveniently connected to conventional fittings for tubing or syringes such as luer lock fittings and the like.

Tubing of the type used to make the microcannula according to the invention can be obtained from suppliers of laboratory wares such as Cole-Parmer, Chicago, Illinois U.S.A.. Wire suitable for the fabrication into the trochar described here in can be obtained from National Standard Company, Santa Fe Springs, California, U.S.A.

The present invention further comprises that microcannula described hereinabove with the trochar described herein above placed in the lumen of the tube forming the microcannula.

When in use to cannulate a small blood vessel or hollow organ the trochar 10 and microcannula 12 are used as a unit. The trochar 10 is placed tightly fitting in the lumen of the microcannula 12 with the trochar tip 11 protruding beyond the microcannula bevel's leading edge 16. The trochar tip 11 is used to pierce the wall of the blood vessel 18. The microcannula bevel's leading edge comes into contact with the outside of the blood vessel 18 outer wall slightly displacing the wall of the blood vessel and stretching the hole punctured in the blood vessel wall. By further advancing the microcannula and trochar together, the microcannula is easily threaded through the hole into the lumen of the blood vessel.

As a result of the tight fit of the trochar and microcannula the cannulation of small blood vessels can be accomplished without the necessity of first incising a blood vessel followed by insertion of a cannula and trochar. By using the trochar and microcannula as a unit, bleeding can be minimized and the possibility of damaging the blood vessel with an incision that is too large is eliminated.

## Claims

1. A microcannula comprising a hollow tube (12) having a cross sectional size smaller than a 24 gauge (0.56 mm) needle and a bevelled tip (16) wherein the angle of said bevel ranges from 23 to 27°, characterised in that the microcannula further comprises a pointed trochar (10, 11) that fits in the lumen of the microcannula tube (12).

2. The microcannula according to claim 1 wherein said bevel is 25° ± 0.50°.

3. The microcannula according to Claims 1 or 2 wherein the outside diameter of said tube is 0.4064 mm ± 0.0254 mm (0.016 inch ± 0.001 inch).

4. The microcannula according to Claims 1, 2 or 3 wherein the inside diameter of said tube is 0.2032 mm ± 0.0254 mm (0.008 inch ± 0.001 inch).

5. The microcannula according to any one of the preceding claims wherein the walls of said tube are a bio-compatible polymer.

6. The microcannula according to claim 5 wherein said bio-compatible polymer is a perfluorocarbon material.

7. The microcannula according to any one of the preceding claims wherein the point of said trochar forms an angle of 8° ± 3°.

8. The microcannula according to claim 7 wherein the length of the point of said trochar is six times the diameter of said trochar.

9. The microcannula according to claims 7 or 8 wherein the outside diameter of said trochar is smaller than the inside diameter of said tube.

10. The microcannula according to claim 9 wherein said trochar is removable from said microcannula but fits tightly in the lumen of said microcannula.

11. The microcannula according to claim 10 wherein said trochar is made of stainless steel.

12. The microcannula according to any one of the preceding claims further comprising a segment (14) distal to the tip of said microcannula with an expanded outside diameter.

13. The microcannula according to claim 12 wherein the expanded outside diameter of said segment is provided by a ring around the outside of said tube.

## Patentansprüche

1. Mikrokanüle mit einem Hohlröhrchen (12), dessen Querschnitt kleiner ist als derjenige einer Injektionsnadel Nr. 24 (0,56 mm) und der eine abgeschrägte Spitze (16) aufweist, wobei der Winkel der Abschrägung zwischen 23 und 27° liegt, dadurch gekennzeichnet, daß die Mikrokanüle weiter einen gespitzten Trokar (10, 11) aufweist, der in den Hohlraum des Mikrokanülenröhrchens (12) paßt.

2. Mikrokanüle nach Anspruch 1, wobei die Abschrägung 25° ± 0,50° beträgt.

3. Mikrokanüle nach Anspruch 1 oder 2, wobei der Außendurchmesser des Röhrchens 0,4064 mm ± 0,0254 mm (0,016 Zoll ± 0,001 Zoll) beträgt.

4. Mikrokanüle nach Anspruch 1, 2 oder 3, wobei der Innendurchmesser des Röhrchens 0,2032 mm ± 0,0254 mm (0,008 Zoll ± 0,001 Zoll) beträgt.

5. Mikrokanüle nach einem der vorhergehenden Ansprüche, wobei die Wände des Röhrchens aus einem biologisch verträglichen Polymer bestehen.

6. Mikrokanüle nach Anspruch 5, wobei das biologisch verträgliche Polymer ein Perfluorkohlenstoffmaterial ist.

7. Mikrokanüle nach einem der vorhergehenden Ansprüche, wobei die Spitze des Trokars einen Winkel von 8° ± 3° bildet.

8. Mikrokanüle nach Anspruch 7, wobei die Länge der Spitze des Trokars sechsmal so groß ist wie der Durchmesser des Trokars.

9. Mikrokanüle nach Anspruch 7 oder 8, wobei der Außendurchmesser des Trokars geringer ist als der Innendurchmesser des Röhrchens.

10. Mikrokanüle nach Anspruch 9, wobei der Trokar aus der Mikrokanüle herausnehmbar ist, wobei er jedoch eng anliegend in den Hohlraum der Mikrokanüle paßt.

11. Mikrokanüle nach Anspruch 10, wobei der Trokar aus rostfreiem Stahl besteht.

12. Mikrokanüle nach einem der vorhergehenden Ansprüche, welche weiter ein distal zur Spitze der Mikrokanüle angeordnetes Segment (14) mit einem erweiterten Außendurchmesser aufweist.

13. Mikrokanüle nach Anspruch 12, wobei der erweiterte Außendurchmesser des Segments durch einen um das Äußere des Röhrchens herum angeordneten Ring bereitgestellt ist.

## Revendications

1. Microcanule comportant un tube creux (12) ayant en coupe une dimension inférieure à celle d'une aiguille de jauge 24 (0,56 mm) et un bout chanfreiné (16) dont l'angle du chanfrein est compris entre 23 et 27°, caractérisée en ce que la microcanule comporte en outre un trocart pointu (10, 11) qui se loge dans la lumière du tube (12) de la microcanule.

2. Microcanule selon la revendication 1, dans laquelle le chanfrein forme un angle de 25 ± 0,50°.

3. Microcanule selon la revendication 1 ou 2, dans laquelle le diamètre externe du tube est égal à 0,4064 mm ± 0,0254 mm (0,016 pouce ± 0,001 pouce).

4. Microcanule selon la revendication 1, 2 ou 3, dans laquelle le diamètre interne du tube est de 0,2032 mm ± 0,0254 mm (0,008 pouce ± 0,001 pouce).

5. Microcanule selon l'une quelconque des revendications précédentes, dans laquelle les parois du tube sont formées d'un polymère biocompatible.

6. Microcanule selon la revendication 5, dans laquelle le polymère biocompatible est un matériau perfluorocarboné.

7. Microcanule selon l'une quelconque des revendications précédentes, dans laquelle la pointe du trocart forme un angle de 8° ± 3°.

8. Microcanule selon la revendication 7, dans laquelle la longueur de la pointe du trocart est égale à six fois le diamètre du trocart.

9. Microcanule selon la revendication 7 ou 8, dans laquelle le diamètre externe du trocart est inférieur au diamètre interne du tube.

10. Microcanule selon la revendication 9, dans laquelle le trocart peut être retiré de la microcanule mais s'ajuste intimement dans la lumière de la microcanule.

11. Microcanule selon la revendication 10, dans laquelle le trocart est formé d'acier inoxydable.

12. Microcanule selon l'une quelconque des revendications précédentes, comprenant en outre un segment (14) distal par rapport au bout de la microcanule qui a un diamètre externe agrandi.

13. Microcanule selon la revendication 12, dans laquelle le diamètre externe agrandi du segment est formé par un anneau placé autour de l'extérieur du tube.
